# EUROPEAN PATENT SPECIFICATION

(11) **EP 4 457 327 B1**
(45) Date of publication and mention of the grant of the patent: **14.10.2026**
(21) Application number: 22840105.5
(22) Date of filing: 19.12.2022
(51) Int. Cl.: C11D 3/48, C11D 1/02, C11D 1/12, C11D 1/28, C11D 3/20, C11D 3/37, C11D 17/00, A61K 8/02, A61K 8/36, A61K 8/368, A61K 8/46, A61K 8/86, A61Q 17/00, A61Q 19/10

(54) **BAR COMPOSITION HAVING ENHANCED ANTIMICROBIAL ACTIVITY**
STABZUSAMMENSETZUNG MIT VERBESSERTER ANTIMIKROBIELLER WIRKUNG
COMPOSITION DE BARRE PRÉSENTANT UNE MEILLEURE ACTIVITÉ ANTIMICROBIENNE

(30) Priority: 27.12.2021 EP 21217786
(43) Date of publication of application: 06.11.2024
(73) Proprietor: Unilever IP Holdings B.V., 6708 WH Wageningen (NL); Unilever Global IP Limited, Wirral, Merseyside CH62 4ZD (GB)
(72) Inventor: CHANDAR, Prem, 6708 WH Wageningen (NL); KUZNITZ, Matthew Fred, 6708 WH Wageningen (NL); VINSKI, Paul, 6708 WH Wageningen (NL); WALSH, Connor Patrick, 6708 WH Wageningen (NL)
(74) Representative: Unilever Patent Group
(86) International application number: PCT/EP2022/086591
(87) International publication number: WO 2023/126228

(56) References cited:
- WO-A1-2021/084308
- CA-C- 2 193 469

## Description

### Field of the Invention

The present invention is directed to a mild bar composition with enhanced antimicrobial activity. More particularly, the bar composition of the present invention is mild and unexpectedly yields superior antimicrobial activity when used in cleansing applications. The bar composition comprises an anionic surfactant and an acid, and is formulated, when lathered, to generate a wash slurry having a pH from 2.9 to 5.2. Such bars may be made via conventional techniques, including extrusion and cast melting techniques, and they may be formulated substantially free of ethanol, parabens, formaldehyde donors, silicones, sulfates and/or halogenated antimicrobial actives.

### Background of the Invention

Cleansing compositions, including bars and liquids, are known to provide many benefits to consumers using them. Germs, in general, can accumulate on hands, hair, cloths and hard surfaces and they may be passed from person to person or object to person via the air and/or routine and ordinary contact. Once on the hands, for example, infection from the germs is inevitable since consumers are constantly touching their eyes, nose and mouth, resulting in a host of illnesses, including respiratory and gastrointestinal illnesses. The Center for Disease Control reports that washing hands with soap removes germs much more effectively than water alone and reduces the chances of getting common aliments like diarrhea and the common cold. In fact, regular use of cleansing compositions reduces the amount of antibiotics people use and the likelihood that antibiotics will become less effective to those in need of them.

Additionally, cleansing compositions traditionally have biocidal action against many Gram negative bacteria. The biocidal action of wash compositions against Gram negative and Gram positive bacteria is considerably much less common, and especially, when the compositions are substantially free of soap, sulfates and halogenated actives, and formulated to be very mild on skin.

There is an increasing interest to develop a bar composition that is mild and yields exceptional antimicrobial activity when used on skin, hair, clothing, hard surfaces or the like. This invention, therefore, is directed to a bar composition that unexpectedly yields superior antimicrobial activity when used in cleansing applications. The bar composition comprises an anionic surfactant and an acid, and is formulated to generate a wash slurry having a pH from 2.9 to 5.2. Such bars may be made via conventional techniques, including extrusion and cast melting techniques, and they may optionally be formulated substantially free of ethanol, parabens, formaldehyde donors, silicones, sulfates and/or halogenated antimicrobial actives.

### Additional Information

CA 2 193 469 C relates to a soap-free cleansing bar, substantially low in fatty acids, and including a synthetic detergent such as sodium cocoyl isethionate, a higher fatty alcohol such as cetyl alcohol, and a processed grain such as colloidal oatmeal.

WO 2021/084308 A1 relates to solid cleansing products that are substantially free of sulfates, are foamable in the presence of a fluid, such as water.

Efforts have been disclosed for making antimicrobial soap bars. In U.S. Patent No. 6,794,344, soap based bar compositions having 50% soap with an alkyl chain length of 8 to 10 carbon atoms are described.

Other efforts have been disclosed for making soap-based formulations. In U.S. Patent Application Publication No. 2010/0098776, disclosed are soap-based liquid wash formulations with enhanced deposition of conditioning and/or skin appearance enhancing agents.

Even other efforts have been disclosed for making soap bar compositions. In WO16024090A1, a semi-translucent soap bar composition with antimicrobial benefit is described.

None of the additional information describes a bar composition having enhanced antimicrobial activity as claimed herein.

### Summary of the Invention

In a first aspect, the present invention is directed to bar composition comprising:
a) from 20 to 60% by weight anionic surfactant;
b) from 0.2 to 8% by weight of at least one acid having a pKa from 1 to 8 and a log P from 0 to 4.95; and
c) bar structurant fatty acid, polyalkylene oxide or mixture thereof

wherein the bar composition in a 10% aqueous slurry has a pH from 2.9 to 5.2, with the proviso that when the slurry has a pH over 4.45 and less than 0.5% by weight of at least one acid having a pKa from 1 to 8 and a log P from 0 to 4.95, the bar composition has less than 3% by weight of the total of betaine, sultaine derived surfactant and mixtures thereof, and further wherein the bar composition is substantially sulfate free substantially free meaning no more than 1.5% by weight of the total weight of the bar composition,
wherein the anionic surfactant comprises a mixture of taurate and isethionate in a weight ratio from 99:1 to 1:99 and the surface is skin.

In a second aspect, invention is directed to method for reducing antimicrobial presence (both Gram negative and Gram positive) on a surface in need of antimicrobial reduction comprising the steps of:
a) contacting the surface with a bar slurry made from the bar composition according to the first aspect of the invention and comprising:
   i) from 60 to 99% by weight water at a temperature of from 32 to 46°C;
   ii) from 1 to 40% by weight soluble and insoluble components from the bar composition; and
b) rinsing the slurry from the surface,
wherein the soluble and insoluble components make up a total component weight in the slurry that comprises from 20 to 40% by weight soluble component in step a) and further wherein the bar composition has an antimicrobial activity in a 10% slurry that provides a Log₁₀ Reduction against *E. coli* ATCC 10536 and *S. aureus* ATCC 6538 of at least 2.75 (preferably 3.0) after 30 seconds of contact with a surface (e.g., skin or a counter top) in need of antimicrobial reduction.

Skin, as used herein, is meant to include skin on the arms (including underarms), face, feet, neck, chest, hands, legs, buttocks and scalp (including hair). Will dissolve means water soluble and is herein defined to mean at least 0.1 to 1 gram of bar composition component (i.e., ingredient), like anionic surfactant, will dissolve in 100ml of water at 25 to 50°C. Component means bar composition ingredient and including water soluble and insoluble ingredients. Aqueous slurry is defined to be a diluted composition which is ready to use (i.e., a diluted end use bar composition) in any cleansing application, and therefore, the same can be a diluted bar composition that is a shampoo, make-up remover, face cleanser or personal wash for body and/or hands. Such an aqueous slurry is also suitable as a home care cleanser (for an inanimate object), like a cleanser for a table, window, sink, toilet or a cleanser for laundry. In an embodiment of the invention, the aqueous slurry is a diluted personal wash composition for skin. In another embodiment, the aqueous slurry is a diluted personal wash composition for the hands. The bar composition of the present invention is suitable to deliver in slurry an active pharmaceutical ingredient (e.g., like corticoids, antihistamines and/or antibiotics). In a preferred embodiment, the bar composition is a cosmetic composition and the aqueous slurry is a cosmetic and non-therapeutic composition suitable to remove soil (water and fat soluble) from skin, and especially, skin on the hands. The aqueous slurry is surprisingly mild while simultaneously delivering antimicrobial benefits. The such a slurry is surprisingly mild while simultaneously delivering antimicrobial benefits. Mild, as used herein, means having a Zein value ≤10 as defined and determined via the method described in the Examples. Antimicrobial benefits means a 10% aqueous slurry providing a Log₁₀ reduction ( temperature at 46°C) against *E. coli* ATCC 10536 and *S. aureus* ATCC 6538 of at least 2.75 (preferably 3.0) after 30 seconds of contact with a surface, like skin, in need of antimicrobial reduction. Bar structurant fatty acid and polyalkylene oxide means a component that contributes to the hardness of the bar composition and/or lather characteristics of the bar composition in aqueous slurry and is typically a solid when at a temperature of less than 40°C. As used herein, pKa is the -log₁₀ Ka where Ka is the acid dissociation constant. Log P is the log₁₀ (Partition Coefficient) where the partition coefficient, P is equal to [Compound]_{octanol}/[Compound]_{water}. Betaine or sultaine derived surfactant means a surfactant with a polar head group that is a betaine or sultaine moiety having a fatty portion where the same may, hereinafter, be referred to as betaines and sultaines.

Substantially free of means no more than 3.5% by weight of the total weight of the bar composition, and preferably, less than 1.5% by weight of the total weight of bar composition. In still another embodiment, substantially free of means less than 1%, and preferably, less than 0.5% by weight of the total weight of the bar composition. Substantially free of, as used herein, is meant to include 0.00001% to 3.5%, and 0.00001 to 1%, and 0.00001 to 0.5%, and 0.0% by weight of the total weight of the bar composition. As to soap, substantially free (of soap) means less than 9%, and preferably, less than 8.5%, and most preferably no more than 8% by weight soap based on total weight of the bar composition. In an embodiment of the invention, the bar composition comprises from 0.00001 to 5.5% or 0.001 to 4.5% by weight soap. In another embodiment, the bar composition comprises less than 2% or less than 1% by weight soap or no (0.0% by weight) soap.

In even another embodiment of the invention, the bar composition can be formulated to be substantially free of ethanol, paraben, formaldehyde donors, sulfates, halogenated antimicrobial actives and any ingredients tested on animals. In another embodiment of the invention, the bar composition comprises less than 1% by weight or 0.0% by weight (based the total weight of the bar composition) of C₁₆₋₁₈ methyl ester sulphonate, glycinate, carboxylate, glutamates and/or alpha olefin sulfonate. In still another embodiment, the bar composition comprises less than 2% by weight silicone or 0.01 to 1.5% or 0.0% by weight silicone based on total weight of the bar composition.

Skin benefit agents can optionally be used in the bar composition of the present invention and such agents may be water or oil soluble. The aqueous slurry will have a viscosity from 20 to 1,500 mPa.s where viscosity is taken with a Brookfield helipath TD, at 4 rpm for 1 minute at 25°C unless noted otherwise. In the absence of explicitly stating otherwise, all ranges described herein are meant to include all ranges subsumed therein. The term comprising is meant to encompass the terms consisting essentially of and consisting of. For the avoidance of doubt, and for illustration, an aqueous slurry comprising water, anionic surfactant and acid is meant to include a slurry consisting essentially of the same and a slurry consisting of the same. Except in the operating comparative examples, or where otherwise explicitly indicated, all numbers in this description indicating amounts or ratios of materials or conditions and/or physical properties of materials and/or use are to be understood as modified by the word "about".

### Detailed Description of the Invention

As to anionic surfactants suitable for use in the bar composition of the present invention, such surfactants can include glycinates, alpha olefin sulfonates, glutamates, aspartates, aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or an alkyl glyceryl ether sulfonate (AGSs) as well as aromatic sulfonates such as alkyl benzene sulfonate.

The anionic surfactant used in the present invention can optionally include some alkyl sulfate (e.g., C₁₂-C₁₈ alkyl sulfate) and/or alkyl ether sulfate (including alkyl glyceryl ether sulfates). As noted herein, the bar composition of the present invention is substantially sulfate free. If used, the sulfates suitable include those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium. As noted, sulfates, when optionally used, make 3.5% or less by weight of the bar composition.

Glycinates like sodium lauroyl glycinate, sodium cocoyl glycinate or mixtures thereof are suitable for use as are glutamates like sodium lauroyl glutamate and/or sodium cocoyl glutamate.

As to alpha olefin sulfonates, the same can optionally be used in the bar composition of the present invention, and C₁₄-C₁₆ olefin sulfonates are generally used when such anionic surfactants are desired.

In an embodiment of the invention, the anionic may also include alkyl sulfosuccinates (including mono- and dialkyl, e.g., C₆-C₂₂ sulfosuccinates); alkyl and acyl taurates (often methyl taurates), alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, or the like.

Sulfosuccinates may be monoalkyl sulfosuccinates having the formula:

R¹O₂CCH₂CH(SO₃M)CO₂M;

and amide-MEA sulfosuccinates of the formula:
R¹CONHCH₂CH₂O₂CCH₂CH(SO₃M)CO₂M wherein R¹ is a C₈-C₂₂ alkyl group.

Sarcosinates suitable for use are generally represented by the formula:
R²CON(CH₃)CH₂CO₂M, wherein R² is a C₈-C₂₀ alkyl group.

Taurates suitable for use are generally identified by formula:

R³CONR⁴CH₂CH₂SO₃M

wherein R³ is a C₈-C₂₀ alkyl group, R⁴ is a C₁-C₄ alkyl group, and M is a solubilizing cation as previously described.

Isethionates suitable for use in the bar composition may include C₈-C₁₈ acyl isethionates (including those which have a substituted head group such as a C₁₋₄ alkyl substitution, preferably methyl substitution). These esters are typically prepared prepare by a reaction between alkali metal isethionate with mixed aliphatic fatty acids having from 6 to 18 carbon atoms and an iodine value of less than 20. Often at least 75% of the mixed fatty acids have from 12 to 18 carbon atoms and up to 25% have from 6 to 10 carbon atoms. Isethionates having at head substituted isomers are described in world application WO 94/09763 and US Patents 2,810,747 and 2,820,818. In an embodiment of the invention, the acyl potion of the isethionate is from 85 to 99%, and preferably, from 92 to 98%, and most preferably, 94 to 97% by weight a single carbon chain length, based on total weight of the acyl portion on the isethionate.

The acyl isethionate that may be used can be an alkoxylated isethionate such as those described in Ilardi et al., U.S. Pat. No. 5,393,466, entitled "Fatty Acid Esters of Polyalkoxylated isethonic acid; issued Feb. 28, 1995. This compound has the general formula:

R⁵C-O(O)-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

Illustrative examples of glutamates suitable for use in the present invention include sodium lauroyl glutamate, sodium cocoyl glutamate, potassium lauroyl glutamate, potassium cocoyl glutamate, disodium cocoyl glutamate, mixtures thereof or the like. Illustrative examples of aspartates suitable for use either alone or together with glutamates and/or other anionic surfactants include sodium lauroyl aspartate, potassium lauroyl aspartate, sodium cocoyl aspartate, potassium cocoyl aspartate, mixtures thereof or the like.

Citrates, including laureth-7 citrate, are suitable for use as anionic surfactants as are carboxylates like sodium laureth-5 carboxylate, sodium lauryl glucose carboxylate, sodium alkyl pareth-8 carboxylates and sodium alkyl pareth-12 carboxylates. Sulfonated tallow fatty acids are yet another class of anionics suitable for use.

Succinates suitable for use in the bar composition of the present invention include disodium laureth sulfosuccinate, disodium lauryl sulfosuccinate, MEA-sulfosuccinate or mixtures thereof. Sarcosinates like sodium and/or potassium lauroyl sarcosinate is/are also suitable for use.

In one embodiment of the invention, the anionic surfactant used is sodium cocoyl isethionate, sodium lauroyl isethionate, sodium myristoyl isethionate, sodium oleoyl isethionate, ammonium oleoyl isethionate or a mixture thereof. Other isethionates suitable for use include acyl alkyl isethionate esters including acyl 1-alkyl, 2-alkyl and 1,2-alkyl isethionates where the alkyl group is often a C₁-C₄-alkyl, and preferably, a C₁ and/or C₂ alkyl, and most preferably, a methyl group. In an embodiment of the invention, a mixture of acyl 1-alkyl and acyl 2-alkyl isethionates in a weight ratio from 1:25 to 1:2, and preferably, 1:20 to 1:3, and most preferably, from 1:18 to 1: 4, respectively, may be used where the alkyl group is a C₁-C₆ alkyl and preferably a methyl and/or ethyl group. In still another embodiment of the invention, the acyl alkyl isethionate is sodium lauroyl methyl isethionate in a mixture where at least 50 to 95%, and preferably, 60 to 85%, and most preferably, 65 to 80% by weight of the methyl group is on carbon two of the isethionate group, based on total weight of the isethionate in the bar composition.

In still another embodiment, the anionic surfactant is a taurate like sodium lauroyl taurate, sodium methyl lauroyl taurate, sodium methyl cocoyl taurate, sodium methyl oleoyl taurate, sodium methyl myristoyl taurate, potassium methyl myristoyl taurate, calcium methyl lauroyl taurate, potassium methyl lauroyl taurate, ammonium methyl lauroyl taurate or a mixture thereof.

In yet another embodiment of the invention, the anionic surfactant used in the bar composition of the present invention is a mixture of any of those described herein.

In, however, another embodiment, the anionic surfactant used is an isethionate, a taurate or a mixture thereof. When a mixture of isethionate and taurate, the mixture is in a taurate to isethionate weight ratio from 99:1 to 1:99, and preferably, from 15:85 to 85:15, and most preferably, from 25:75 to 75:25 based on total weight of taurate and isethionate in the bar composition. Still in another embodiment, the weight ratio of taurate to isethionate in the bar composition is 35:65 to 65:35 or 58:42 to 42:58 based on total weight of taurate and isethionate in the bar composition.

Often, and in another embodiment, the anionic surfactant in the bar composition of the present invention is at least 80% by weight isethionate and/or taurate, and preferably, at least 90% by weight isethionate and/or taurate, and most preferably, at least 95 to 100% by weight isethionate and/or taurate based on total weight of the anionic surfactant in the bar composition. A bar composition with anionic surfactant at 100% by weight isethionate or 100% by weight taurate (based on total weight of anionic surfactant in the bar composition) is also within the scope of the present invention. It is also within the scope of the present invention for the bar composition to comprise only anionic surfactant (i.e., 100%) as the surfactant in the composition, and therefore, a bar composition with anionic surfactant and 0.0% by weight betaine is within the scope of the invention.

Anionic surfactant typically makes up from 18 to 65%, and preferably, from 20 to 60%, and most preferably, from 25 to 55% by weight of the bar composition. In an embodiment of the invention, anionic makes up from 23.5 to 35.5% or 26.5 to 33.5% by weight of the bar composition.

When optionally desired, amphoteric surfactants suitable for use can be used in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate or mixtures thereof. If present such surfactants make up from 0.00001 to 6.0%, and preferably, from 0.1 to 4.5%, and most preferably, from 0.1 to 2.5% by weight of the bar composition. The bar composition can comprise 0.00001 to 1% by weight amphoteric surfactant and no (0.0%) by weight of the bar composition.

As to zwitterionic surfactants that may optionally be employed in the present invention, such surfactants include at least one acid group. Such an acid group may be a carboxylic or a sulphonic acid group. They often include quaternary nitrogen, and therefore, can be quaternary amino acids. They often generally include an alkyl or alkenyl group of 7 to 18 carbon atoms generally comply with an overall structural formula:
R⁶-[--C(O)-NH(CH₂)₄--]-N⁺--(R⁷--)(R⁸)A-B where R⁶ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is -CO₂-or -SO₃--.

Suitable zwitterionic surfactants for optional use in the present invention do include simple betaines of the formula:

R⁶-N⁺--(R⁷)(R⁸)CH₂CO₂⁻

and amido betaines of the formula:
R⁶-CONH(CH₂)ₜ-N⁺--(R⁷)(R⁸)CH₂CO₂⁻ where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

Another option is that the zwitterionic surfactant is a sulphobetaine of the formula:

R⁶-N⁺--(R⁷)(R⁸)(CH₂)₃SO₃⁻

or

R⁶-CONH(CH₂)ᵤ-N⁺--(R⁷)(R⁸)(CH₂)₃SO₃⁻

where u is 2 or 3, or variants of these in which -(CH₂)₃SO₃⁻ is replaced by - CH₂C(OH)(H)CH₂SO₃⁻.

In these formulae, R⁶, R⁷ and R⁸ are as previously defined.

Illustrative examples of the zwitterionic surfactants suitable for use include betaines like coco betaine, cocoamidopropyl betaine, lauryl betaine, laurylhydroxy sulfobetaine, lauryldimethyl betaine, cocoamidopropylhydroxylsulfo betaine, behenyl betaine, capryl/capramidopropyl betaine, cocodimethyl carboxymethyl betaine, stearyl betaine.

laurylamidopropyl betaine, mixtures thereof or the like. An additional zwitterionic surfactant suitable for use includes lauryl hydroxysultaine and/or cocamidopropyl sultaine. In a preferred embodiment, the zwitterionic surfactant used in the bar composition of this invention is cocamidopropyl betaine.

Zwitterionic surfactants, when used, may make up from 0.0001 to 8%, and preferably, from 1.0 to 6%, and most preferably, from 2 to 5% by weight of the bar composition. The bar composition can comprise from 0.0001 to 3.5% by weight zwitterionic surfactant, or 2.5 to 3.5 % by weight of the bar composition or no (0.0% by weight) zwitterionic surfactant. When the pH of a 10% wash slurry is over 4.45, the bar composition of the present invention has less than 3%, and preferably, less than 2%, and most preferably, less than 1% by weight of the total of betaine and sultaine derived surfactant and mixtures thereof, based on the weight of the bar composition. In an embodiment of the invention the total zwitterionic surfactant, like betaine and/or sultaine, when used in the invention will make up from 0.05 to 10%, and preferably, from 0.08 to 8%, and most preferably, from 0.5 to 6.5% by weight of the total weight of zwitterionic and anionic surfactant used in the bar composition. In still another embodiment, the total of betaine and/or sultaine make(s) up from 1 to 2.5% and anionic surfactant makes up from 26 to 36% by weight of the bar composition. In another embodiment, the total of betaine and/or sultaine derived surfactant (in total) make(s) up from 0.5 to 2.75% and anionic surfactant makes up from 26.5 to 35% by weight of the bar composition. In still another embodiment, when betaine and/or sultaine are used in the bar composition of the present invention, the anionic surfactant comprises less than 7%, and preferably less than 5%, and most preferably, less than 4% by weight taurate based on total weight of anionic surfactant in the bar composition. In yet another embodiment, when betaine and or sultaine is/are used, the anionic surfactant comprises from 0.001 to 3.5% by weight taurate based on total weight of the anionic surfactant or no taurate (0.0% by weight).

Nonionic surfactants may optionally be used in the bar composition of the present invention. When used, nonionic surfactant typically is included at levels as low as 0.5, 1, 1.5 or 2% by weight and at levels as high as 6, 8, 10 or 12% by weight of the bar composition. The nonionics which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

Nonionic surfactants optionally used can include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

In an embodiment of the invention, cationic surfactants may optionally be used in the bar composition of the present invention. One class of optional cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for optional use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other optional cationic surfactants suitable for optional use include quaternized hydrolysates of silk, wheat, and keratin proteins, and it is within the scope of the invention to use mixtures of the aforementioned cationic surfactants.

If used, cationic surfactants will make up no more than 0.75% by weight of the bar composition. When present, they typically make up from 0.001 to 0.7%, and more typically, from 0.01 to 0.5% or 0.0% by weight of the bar composition.

In an embodiment of this invention, the bar composition of this invention may optionally comprise from 0.0001 to 0.5% by weight polymeric quaternary ammonium compounds (including salts of the same). In another embodiment, the bar composition will comprise less than 0.3% by weight polymeric quaternary ammonium compounds. In yet another embodiment, the bar composition comprises less than 0.01% by weight polymeric quaternary ammonium compounds. In even another embodiment, the bar composition is free of polymeric quaternary ammonium compounds (i.e., 0.0% by weight).

The surfactants suitable for use in the present invention are available from suppliers like Stepan Company, Clariant AG, Croda, Galaxy Surfactants, Sino Lion, Innospec, Dow Chemical and the like.

As to the acid suitable for use in the present invention, the same is limited only to the extent that it is suitable for use in a consumer product, has a pKa from 1 to 8 and a log P from 0 to 4.95. Such an acid is meant to include acids that are mono- and dicarboxylic acids, unsaturated acids, aromatic acids, phosphonic acids or mixtures thereof.

In an embodiment of the invention the acid (or mixture of acids used) has a pKa from 1.5 to 7, and preferably, from 2 to 6.5, and most preferably, from 2 to 5.5. In another embodiment of the invention, the pKa of the acid used is from 2.5 to 4.8. In still another embodiement, the pKa is from 2.75 to 3.25. In one embodiemnt of the invention, the log P of the acid or acids used is from 0.65 to 4, and preferably, from 1 to 3.3, and most preferably, from 1.2 to 3. In yet another embodiment, the log P of the acid is 1.4 to 2.8 or 1.6 to 2.6.

As to the carboxylic acids suitable for use, illustrative examples include propionic, n-butryic, isobutryic, pentanoic, hexanoic, heptanoic, octanoic, nonanoic and/or decanoic acid. Dicarboxylic acids suitable for use include adipic and/or pimelic acid. The carboxylic acids can also include those which are unsaturated like acrylic, but-3-enoic, but-2-ynoic, crotonic, isocrotonic, sorbic, angelic, tiglic, and/or tetrolic acid. It is also within the scope of the invention to use or include with other acids those classified as aromatic acids. The aromatic acids suitable for use include coumaric, benzoic, p-amino benzoic, salicylic, capryloyl salicylic, 3-hydroxybenzoic, 4-hydroxybenzoic and/or gallic acid. Phosphonic acids suitable for use include 4-ethoxyphenylphosphonic, 4-methylphenylphosphonic, 4-chlorophenylphosphonic, phenylphosphonic acid or a mixture thereof.

It is within the scope of the invention to include any combination of the acids having a pKa from 1 to 8 and a log P from 0 to 4.75 as described herein. In an embodiment of the invention, the acid used is benzoic, p-aminobenzoic, capryloyl salicylic acid and/or salicylic acid. In another embodiment, the acid used comprises salicylic acid and benzoic acid at a weight ratio of 5:95 to 95:5, and preferably, from 25:75 to 75:25, and most preferably, from 40:60 to 60:40. In another embodiemnt of the invention, the acid used consists essentially of salicylic acid and benzoic acid at a weight ratio of 5:95 to 95:5, and preferably, from 25:75 to 75:25, and most preferably, from 40:60 to 60:40. In even another embodiment of the invention, the acid used is salicylic acid and benzoic acid at a weight ratio of 5:95 to 95:5, and preferably, from 25:75 to 75:25, and most preferably, from 40:60 to 60:40.

In still another embodiment of the invention, the acid used is all (100% by weight of the acid having a pKa from 1 to 8 and a log P from 0 to 4.75) benzoic acid or all salicylic acid.

Regarding the amount of such acids used, typically from 0.2 to 8%, and preferably from 0.2 to 7%, and most preferably, from 0.25 to 6.5% by weight of acid is used based on total weight of the bar composition. In an embodiment of the invention, from 0.275 to 5.75% by weight of acid is used. In even another embodiment, from 0.325 to 5.5% by weight of acid is used based on total weight of bar composition.

In an embodiment of the invention, the amount of soap used is from in the bar composition of the present invention is from 1.1 to 2.4 or from 1.2 to 2.3 or from 1.2 to 2.2 times the amount of acid having a pKa from 1 to 8 and a log P from 0 to 4.95 used in the invention with the proviso that the amount of soap does not exceed 9% by weight of the bar composition.

As to the bar structurant fatty acid used to build or form the bar composition of the present invention, the same is often a blend of C₈ to C₁₈, preferably C₁₂ to C₁₈, and most preferably, C₁₆ to C₁₈ fatty acids.

Water soluble polyalkylene oxide structurants may also be included as part of the composition, as structurant, with or without fatty acid structurant. The water-soluble structurant should melt in the temperature range from 40° C. to 100° C. so that it is suitable for melting to form the bar composition but will be a solid at temperatures at which the bar will be used. Preferably, the structurant has a melting point of at least 50° C. to 90° C. Materials which are suitable for use as the water-soluble structurant are moderately high molecular weight polyalkylene oxides having the noted melting points and in particular polyethylene glycols or mixtures thereof. Polyalkylene glycols (especially polyethylene glycols or PEGs) which may be used typically have a weight average molecular weight in the range 1,500-10,000 (preferably, 7,000 to 9,000). In an embodiment of the invention, it is desired to include a fairly small quantity (less than 10% of total structurant) of polyethylene glycol with a weight average molecular weight in the range from 50,000 to 500,000, especially molecular weights of 90,00 to 110,000. In an embodiment of the invention, bar structurant is not all polyalkylene oxide whereby the same when intended to be part of the bar composition is present at a weight ratio from 3:1 to 2:1, and preferably, 1.8:1 to 1.35:1 of polyalkylene oxide to fatty acid structurant. In an embodiment of the invention, the bar structurant is 15 to 100%, or 30 to 90% or 45 to 70% by weight fatty acid based on total weight of bar structurant used. In even another embodiment, the bar structurant is 100% by weight fatty acid, and especially stearic acid or a stearic acid mixed with 1 to 20% by weight lauric acid based on total weight of stearic and lauric acid in the bar composition.

Typically, bar structurant fatty acid and/or water soluble polyalkylene oxide makes up from 8 to 64%, or 8 to 45%, and preferably, from 10 to 40%, (or 10 to 41%), and most preferably, 20 to 40% (or 20 to 41%) by weight of the bar composition. In yet another embodiment, the bar composition comprises from 21 to 39% or 27 to 37% by weight structurant based on total weight of the bar composition.

Regarding soaps suitable for optional use in the bar composition of the present invention, the same can include insoluble soap (i.e., brick-like) that typically consist of higher chain C₁₆ to C₁₈ soaps such as sodium or potassium soaps of palmitic or stearic acid (palmitate and stearate soap). The same are generally included in the bar composition of the present invention to contribute to structuring benefits that ensure bar body strength, allowing, for example, for shape maintenance of the bar composition. The bar composition of the present invention may also consist of water-soluble fatty soaps (mortar-like) which are traditionally unsaturated C18:1 and/or 18:2 sodium or potassium soaps (oleate soap) in combination with short chain fatty acids (generally C8 to C14 soaps).

Again, when used, salts of carboxylic acids (i.e., soaps) typically make up from less than 9.0%, and preferably, less than 8.5%, and most preferably no more than 8% by weight soap based on total weight of the bar composition. In an embodiment of the invention, the bar composition comprises from 0.00001 to 5.5% or 0.001 to 4.5% by weight soap. In another embodiment, the bar composition comprises less than 2% or less than 1% by weight soap or no (0.0% by weight) soap based on total weight of the bar composition.

Water typically makes up from 0.0 to 13%, and preferably, from 2 to 12%, and most preferably, from 3 to 10% by weight of the bar composition. In an embodiment of the invention, water makes up from 3 to 9% or 4 to 7% by weight of the bar composition.

Regarding the wash slurry, the pH of the same is from 2.9 to 5.2, and preferably, from 3.3 to 4.9, and most preferably, from 3.9 to 4.8. In another embodiment, the pH of the wash composition is from 3.9 to 4.55 or from 3.9 to 4.45 or from 4.0 to 4.42. The wash slurry has viscosity from 20 to 1,500 mPa.s, and preferably, from 30 to 1,300 mPa.s, and most preferably, from 100 to 800 mPa.s. Again, when the wash slurry has a pH over 4.45 and the bar composition has less than 0.5% by weight of at least one acid having a pKa from 1 to 8 and a log P from 0 to 4.95, the bar composition has less than 3% by weight betaine and/or sultaine derived surfactant.

Optional ingredients that may be used in the bar composition of the present invention include preservatives to assist against the growth of potentially harmful microorganisms when the end use wash composition is made. Suitable traditional preservatives that may be used include propionate salts, and a variety of quaternary ammonium compounds. Often preferred preservatives are potassium sorbate, iodopropynyl butyl carbamate, phenoxyethanol, wasabi-based preservatives, imidazolidinyl urea, sodium dehydroacetate and benzyl alcohol. Often desired additives suitable to be employed in the bar composition of the present invention are 1,2-alkanediols like 1,2-octanediol, 1,2 hexanediol or mixtures thereof. The bar composition may comprise of a preservative system that is formaldehyde-free, paraben-free, or both, but it preferably preservative free, especially when the bar comprises less than 12% by weight or less of water.

Traditional fragrance components like eugenol, coumarin, linalyl acetate, citronellal, iris concentrate, terpinyl acetate, terpineol, thymol, pinenes (e.g., alpha and beta pinene) and citronellol may optionally be added to the bar composition. Such components make up from 0.01 to 1.2%, and preferably, from 0.1 to 1% by weight of the bar composition when used.

If employed, the traditional preservatives, vicinal diol and/or fragrance component will not make up more than 2%, and preferably, not more than 1%, and most preferably, from 0.2 to 0.85% by weight of the bar composition. In an embodiment of this invention from 0.2 to 0.8% by weight optional preservative, vicinal diol and/or fragrance component is used, based on total weight of the bar composition. In an embodiment of the invention, no preservative, vicinal diol and/or fragrance component (except for what may be provided in the fragrance used in the bar composition) is used in the bar composition.

Other optional ingredients suitable for use include zinc pyrithione, octopirox, or a mixture thereof, especially when the bar composition is used as a shampoo that provides antidandruff benefits. Each of these substances may range from 0.05 to 3%, preferably between 0.1 and 2% by weight of the total weight of the bar composition.

Additional optional ingredients that may be used include sensory oils and/or exfoliants. Desirable oils include rose, lime, coconut, lavender, argan, sweet almond oil(s) or mixtures thereof. Illustrative exfoliants desirable for use include salt, sugar, apricot, walnut shell, rice, nutmeg and/or oatmeal powder(s). When used, sensory oils and exfoliants can make up from 0.1 to 2% by weight of bar composition, with the proviso that the total amount of fragrance and sensory oil does not exceed 2.5% by weight of the composition, and preferably, not more than 2.0 percent by weight of the composition.

The bar composition may include vitamins. Illustrative vitamins include Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present may range from 0.001 to 5%, and preferably from 0.01% to 3%, optimally from 0.1 to 1% by weight of the composition.

Other optional additives suitable for use include resorcinols like 4-ethyl resorcinol, 4-hexyl resorcinol, 4-phenylethyl resorcinol, dimethoxytoluyl propyl resorcinol, 4-cyclopentyl resorcinol, 4-cyclohexylresorcinol, thiamidol; alpha- and/or beta-hydroxyacids; retinoic acid and its derivatives (e.g., cis and trans); retinal; retinol; retinyl esters such as retinyl acetate, retinyl palmitate, and retinyl propionate; petroselinic acid; conjugated linoleic acid; 12-hydroxystearic acid; mixtures thereof or the like. Conditioning agents such as polyquaternium compounds (e.g., polyquaternium-67) may also be desirable for inclusion in the inventive composition. Such additives, when used, collectively make up from 0.001 to 7%, preferably, from 0.01 to 6%, and, most preferably, from 0.1 to 5% by weight of the bar composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Amounts of these materials when present may range from 0.01 to 3%, and, preferably, from 0.1 to 2% by weight of the bar composition.

A variety of herbal extracts may optionally be included in the cosmetic wash compositions of this invention. Illustrative extracts include those removed from green tea, yarrow, ginseng, marigold, hibiscus, ginko biloba, chamomile, licorice, aloe vera, grape seed, citrus unshiu, willow bark, sage and rosemary. Humectants like glycerol and other polyols (e.g., sorbitol) may also be included. Humectants and/or extracts, such as sorbitol, when used, typically make up from 0.01 to 5%, preferably, from 0.01 to 4%, and most preferably, from 0.02 to 3% by weight of the bar composition.

Another optional additive suitable for use includes hemp oil with 2.5 to 25% by weight cannabigerol and/or cannabidiol at from 0.5 to 10 percent by weight. When used, such oil makes up from 0.0001 to 5% by weight of the composition, and preferably, from 0.01 to 3% by weight of the bar composition.

Also, optionally suitable for use include materials like chelators (e.g., EDTA), opacifiers (like TiO₂, particle size from 50 to 1200 nm, and preferably, 50 to 350 nm), kaolin, bentonite, zinc oxide, iron oxide, mica, C₈₋₂₂ fatty acid substituted saccharides, lipoic acid, retinoxytrimethylsilane (available from Clariant Corp. under the Silcare 1M-75 trademark), dehydroepiandrosterone (DHEA) or mixtures thereof. Ceramides (including Ceramide 1, Ceramide 3, Ceramide 3B and Ceramide 6) as well as pseudoceramides may also be optionally included as can 10- and/or 12-hydroxystearic acid. Amounts of these additional and optional materials, when used, may range from 0.0001 to 3%, and preferably, from 0.001 to 2%, and most preferably, from 0.001 to 1.5% by weight of the bar composition.

Colorants or dyes may also be included in the bar compositions. These substances may range from 0.05 to 5%, and preferably, between 0.1 to 2% by weight of the composition if used.

Conditioning agents like hydroxypropyltrimonium chloride, 5-ureidohydantoin and/or glyoxyldiureide may be used. The components, when used, make up from 0.5 to 4%, and, preferably, from 0.75 to 3%, and most preferably, from 1 to 2% by weight of the bar composition.

Sunscreen actives may also be optionally included in the bar composition of the present invention. Particularly preferred are such materials as ethylhexyl p-methoxycinnamate, available as Parsol MCX^{®}, Avobenzene, available as Parsol 1789^{®} and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.01 to 3%, and preferably, from 0.5 to 2%, optimally, from 0.75 to 1.5% by weight of the bar composition.

Conventional buffers/pH modifiers may be used. These include commonly employed additives like sodium hydroxide, potassium hydroxide, ammonium hydroxide, hydrochloric acid, citric acid/citrate buffers, triethanolamine, or mixtures thereof where pH is determined using a Thermo Fisher Scientific pH meter.

Optional antimicrobial ingredients may be used but are not required. Illustrative ingredients include quaternary ammonium compounds like cetylpyridinium chloride, cetyltrimethylammonium chloride, cetyltrimethylammonium bromide, benzalkonium chloride, benzethonium chloride, cetrimide or mixtures thereof. Certrimonium chloride and/or bromide, chloroxylenol, antimicrobial lipids, antimicrobial peptides or mixtures thereof may also be used. Triclosan may also be used but is not generally desired. Mixtures of thymol and terpineol are often desired for use, typically at a weight ratio from 1:3 to 3:1 or from 1:2 or 2:1 or from 1:0.7 to 0.7:1. When used, such optional antimicrobial ingredients make up from 0.001 to 6%, and preferably, from 0.01 to 5%, and most preferably, from 0.01 to 2.5% by weight of the bar composition.

When preparing bar composition conventional and art recognized extrusion and cast melting techniques may be used. A wide variety of packaging can be employed with the bar composition of this invention. Boxes, bags, recycled raps, metallic containers as well as plastic containers may be used. Preferably, the packaging used with the bar composition of the present invention is packaging made from recycled material like post-consumer resins or biodegradable material. Most preferably, the bar composition is sold in biodegradable packaging material, like paper or cardboard material. In an especially preferred embodiment, the bar composition is used by the consumer directly from the biodegradable packaging.

When washing a surface, including skin or hair, with the aqueous slurry created with the bar composition of the present invention, water is from 32 to 46°C, and preferably, from 34 to 44°C, and most preferably, from 35 to 42°C. Rubbing the bar composition in the hands with water or against the desired surface with water is the typical means for making the aqueous slurry. The aqueous (or bar) slurry will typically comprise from 60 to 99%, or from 70 to 97%, or from 75 to 95%, or from 80 to 92% by weight water. The aqueous slurry will also comprise from 1 to 40%, or from 5 to 30%, or from 6 to 15% by weight soluble and insoluble components from the bar composition. Washing should last for at least 10 seconds, and preferably, for 20 seconds or more, and most preferably, at least 30 seconds.

The following examples are provided to facilitate an understanding of the present invention. The examples are not intended to limit the scope of the claims.

### Examples

Aqueous slurries were prepared whereby gratings from the bars made in the Samples of Table I were mixed with water to form a test solution containing 10% by weight of the initial bar component. In the second method, raw bar components, as depicted in the Samples in Table II, were mixed with water to form a test solution containing a total of 10% raw bar component to simulate an aqueous slurry of a bar composition. For each sample, aqueous slurries were mixed at 50°C to simulate customary consumer washing conditions.

As to the microbes, *E. coli* ATCC 10536 and *S. aureus* ATCC 6538 were used in this study to represent Gram negative and Gram positive bacteria, respectively. The bacteria were stored at -80°C. Fresh isolates were cultured twice on Tryptic Soy Agar plates for 24 hours at 37°C before each experiment.

Test solutions were prepared. In the first method and for the aqueous slurries made from the bar compositions described in Table I, a composition (typically containing up to 15% surfactant) was mixed with water to form a test solution containing 10% weight percent of the initial formulation. In the second method and for the raw ingredients combined in Table II, the same were mixed with water at levels that simulate a 10% dilution of the slurries. In each assessment, test solutions were heated and mixed at 70°C. Test solutions were allowed to cool to room temperature (25°C overnight) prior to testing.

Time-kill assays were adapted from the ASTM International testing method ASTM E2315-16 entitled "Standard Guide for Assessment of Antimicrobial Activity Using a Time-Kill Procedure",. Following this procedure, growth-phase bacterial cultures at 1.5x10⁸ to 5x10⁸ colony forming units per mL (CFU/mL) were treated with the test solutions (prepared as described in the Tables) at 46°C. Eight (8) parts by weight of the 10% test samples, prepared as described above, were combined with 1 part by weight of bacteria culture and 1 part by weight of water. After 30 seconds of exposure, the resulting mixtures were neutralized to arrest the antimicrobial activity of the test solutions. The neutralized bacteria solutions were serially diluted, plated on solid medium, incubated for 24 hours, and viable cells were enumerated through colony counting. Antimicrobial activity is defined as the log reduction in CFU/mL relative to the bacterial concentration at 0 seconds. Assessment time was 30 seconds and a Log₁₀ reduction of 2.75 or higher was acceptable and a pass. Cultures not exposed to any test solutions (i.e., no acid as described or conventional antimicrobial active) served as no-treatment controls.

The log reduction was calculated using the formula:${Log}_{10} Reduction = {Log}_{10} \left(no-treatment control CFU\right) - {Log}_{10} \left(test sample CFU\right) .$

### Example I

Low water content bar compositions, Samples 1-7, were made with the components listed in Table I below. Synthetic detergent and pre-made ribbons were melted and subjected to blow mold extrusion or cast melting processing steps in order to produce the bar compositions described. Log₁₀ Reduction was measured after 30 seconds.

**Table I**

| Ingredient | Weight Percent | | | | Weight Percent (Controls) | | |
|---|---|---|---|---|---|---|---|
| | 1 | 2 | 3 | 4 | 5 | 6 | 7 |
| Taurate | 30 | 30 | 15 | 3 | 30 | 15 | -- |
| SCI* | -- | -- | 15 | 45 | -- | 15 | 45 |
| Betaine | -- | -- | -- | -- | -- | -- | 3 |
| Stearic Acid | 25 | 21.34 | 24.5 | 34.77 | 19.34 | 24.6 | 34.4 |
| Lauric Acid | 1.5 | 1.5 | 2.6 | 5.64 | 1.5 | 2.56 | 5.5 |
| PEG 8000 | 37.4 | 32 | 36.7 | -- | 29 | 36.9 | -- |
| Sodium Stearate | -- | 5 | -- | -- | 10 | -- | -- |
| Sodium Isethionate | -- | -- | 0.55 | 5.81 | -- | 0.5 | 5.8 |
| Water | 5 | 5 | 5 | 5 | 5 | 5 | 5.1 |
| TiO2 | 0.08 | 0.16 | 0.16 | 0.08 | 0.16 | 0.16 | 0.08 |
| Salicylic Acid | 1 | 5 | 0.5 | 0.5 | 5 | 0.25 | 0.5 |
| pH | 3.69 | 3.72 | 4.04 | 4.16 | 5.42 | 5.25 | 4.46 |

### Log₁₀ Reduction

| | | | | | | | |
|---|---|---|---|---|---|---|---|
| S. auerus | 3.73 | 3.73 | 3.46 | 2.77 | 0.07 | 0.27 | 0.9 |
| E. coli | 3.51 | 3.51 | 3.61 | 3.5 | 3.62 | 3.92 | 3.5 |
| | | | 4.91** | | | | |
| | | | 5.01** | | | | |

| | | | | | | | |
|---|---|---|---|---|---|---|---|
| * Sodium cocoyl isethionate ** Test conditions at 40°C | | | | | | | |

As can be seen from the data, aqueous slurries prepared from bar compositions consistent with the present invention (Samples 1-4) unexpectedly resulted in excellent antimicrobial reduction. The aqueous slurries were also assessed/used by skilled panelists, all of which concluded the compositions were mild on the skin, notwithstanding the pH of the slurries being below the natural pH of skin.

### Example II

Samples 8-15 of Table II, again, were made as aqueous slurries with bar composition components. The amounts presented are in weight percent weight based on total weight of aqueous slurry which reflects a slurry made from water and 10% bar composition.

**Table II**

| Ingredients | 8 | 9 | 10 | C.E. 11 | 12 | C.E. 13 | C.E. 14 | C.E. 15 |
|---|---|---|---|---|---|---|---|---|
| | | | | | | | | |
| Taurate | 0.48 | 0.48 | 0.47 | 0.46 | 0.45 | | 0.47 | |
| SCI* | 4.04 | 4.03 | 4.04 | 4.05 | 4.06 | 0.45 | 4.05 | 0.48 |
| Betaine | | | | | | 4.06 | | 4.06 |
| 2:3 weight ratio stearic acid:PEG 8000 | 4.50 | 4.52 | 4.51 | 4.52 | 4.51 | 4.50 | 4.50 | 4.53 |
| Salicylic Acid | 0.10 | | | | | 0.10 | | |
| Benzoic Acid | | 0.10 | | | | | | |
| Phenylpropionic Acid | | | 0.10 | | | | | |
| Lactic acid | | | | 0.10 | | | | |
| C8 Acid (octanoic acid) | | | | | 0.10 | | | |
| Water | 90.87 | 90.86 | 90.88 | 90.86 | 90.87 | 90.90 | 90.98 | 90.93 |
| pH | 4.25 | 4.28 | 4.35 | 4.22 | 4.20 | 4.03 | 4.23 | 4.21 |
| E. coli Log Reduction (46°C, 30 sec) | >3.51 | >3.51 | >3.51 | >3.51 | >3.51 | >3.51 | >3.51 | >3.51 |
| S. aureus Log Reduction (46°C, 30 sec) | >3.52 | 3.20 | 3.22 | 2.25 | 3.28 | 0.01 | 2.39 | 0.00 |

| | | | | | | | | |
|---|---|---|---|---|---|---|---|---|
| * Sodium cocoyl isethionate C.E. Comparative example. | | | | | | | | |

As can be seen from the data, aqueous slurries prepared to reflect diluted bar compositions consistent with the present invention (Samples 8, 9, 10 and 12) unexpectedly resulted in excellent antimicrobial reduction. The aqueous slurries were also assessed/used by skilled panelists, all of which concluded the compositions were mild on the skin, notwithstanding the pH of the slurries being below the natural pH of skin. A log reduction of at least 2.7 after 30 seconds of contact was realized whereas lactic acid which does not satisfy the criteria of the invention performed poorly against S. aureus.

### Example III

Bar formulations A-D were prepared with the noted ingredients and evaluated for antimicrobial efficacy via the method above, and for skin mildness as defined in this Example. The results for the bar composition consistent with the invention were compared to commercially available bar compositions.

### Zein Mildness Assay

Zein Mildness of the aqueous slurries was assessed by combining 0.3g of Zein powder (Sigma. 23625-1kg, lot# SLBZ2926 or Acros Organic^{™} Zein) with 10 ml of a 1% dilution in water of bar composition. The resulting combination was mixed for 30 minutes at 25°C. The resulting mixtures were visually inspected to determine if excess undissolved Zein was present. In cases where excess Zein was not present, an additional 0.3g of Zein was added to the mixtures and further mixed for an additional 30 minutes. The resulting mixtures were then filtered using a 0.45 micron filter. The filtrate was diluted in a 2% sodium dodecyl sulfate solution and the absorbance at 298 nm was measured to determine the amount of dissolved Zein. A control experiment with 1% corresponding bar composition deplete of Zein was prepared in a similar way and measured at 298 nm to measure background. The amount of dissolved Zein is reported as:
Zein Absorbance = [Absorbance (298 nm) of Zein of liquid composition - Absorbance (298 nm) of background composition] * dilution factor. Milder products dissolve less Zein and give lower absorbance values. The range of Zein absorbance values that enable a ranking of mildness of products in terms of ability to irritate skin are defined and described in Table III below.

**TABLE III**

| Zein absorbance values for bar compositions | | | | | |
|---|---|---|---|---|---|
| | | | | | Zein Absorbance Values |
| Mild/minimal or no skin irritation | | | | | ≤10 |
| Moderately harsh/some irritation | | | | | >10 to ≤20 |
| Harsh/high skin irritation | | | | | >20 |

| Ingredient | A | B | C | C.E. D | Dial Gold Antibacterial Soap Bar |
|---|---|---|---|---|---|
| Taurate | 30 | 15 | 30 | 30 | -- |
| SCI* | -- | 15 | -- | -- | -- |
| Betaine | -- | -- | -- | -- | -- |
| Stearic Acid | 20.34 | 24.5 | 20.1 | 19.34 | -- |
| Lauric Acid | 1.5 | 2.6 | 1.5 | 1.5 | -- |
| PEG 8000 | 30.5 | 36.7 | 30.1 | 29 | -- |
| Sodium Soap (Stearate or palmate) | 7.5 | -- | 8 | 10 | -- |
| Sodium Isethionate | -- | 0.55 | -- | -- | -- |
| Water | 5 | 5 | 5 | 5 | -- |
| TiO2 | 0.08 | 0.16 | 0.16 | 0.16 | -- |
| Salicylic Acid | 5 | 0.5 | 5 | 5 | -- |
| | | | | | |
| pH | 4.12 | 4.04 | >3.7 | 5.42 | 10 |
| S.Aureus @ 30s | >3.7 | 3.46 | >3.7 | 0.07 | 0.3 |
| E. Coli @ 30s | >3.7 | 3.61 | 3.62 | 3.62 | >3.7 |
| Zein Absorbance | 5.6 | 5.4 | 5.7 | 6.5 | 27.52 |
| | | | | | |

The data in Table III unexpectedly shows that inventive formulations A-C made consistent with the invention unexpectedly show excellent antimicrobial efficacy while also being surprising mild to skin. Example D, not made consistent with the invention, showed that mildness was achieved but with significant compromise in antimicrobial efficacy especially against gram positive microbes. In comparison, to a commercially available bar composition, the data unexpectedly shows that formulations A-C provided surprising and unexpected benefits of being mild to skin yet excellent for antimicrobial benefits.

## Claims

1. A bar composition comprising:
a) from 20 to 60% by weight anionic surfactant;
b) from 0.2 to 8% by weight of at least one acid having a pKa from 1 to 8 and a log P from 0 to 4.95;
c) bar structurant fatty acid, polyalkylene oxide or mixture thereof
wherein the bar composition in a 10% aqueous slurry has a pH from 2.9 to 5.2, with the proviso that when the slurry has a pH over 4.45 and the bar composition has 0.5% by weight or less of at least one acid having a pKa from 1 to 8 and a log P from 0 to 4.95, the bar composition has less than 3% by weight of total of betaine , sultaine derived surfactant and mixtures thereof, and further wherein the bar composition is substantially sulfate free, substantially free meaning no more than 1.5% by weight of the total weight of the bar composition,
wherein the anionic surfactant comprises a mixture of taurate and isethionate in a weight ratio from 99:1 to 1:99 and the surface is skin.

2. The bar composition according to claim 1, wherein the acid has a pKa from 1.5 to 7, or
from 2.5 to 4.8 or from 2.75 to 3.25 and a log P from 0.65 to 4 or from 1 to 3.3 or from 1.2 to 3.

3. The bar composition according to any one of the preceding claims, wherein the acid is propionic, n-butryic, isobutryic, pentanoic, hexanoic, heptanoic, octanoic, nonanoic, decanoic, pimelic, but-3-enoic, but-2-ynoic, crotonic, isocrotonic, sorbic, angelic, tiglic, tetrolic, coumaric, benzoic, p-amino benzoic, salicylic, capryloyl salicylic, 3-hydroxybenzoic, 4-hydroxybenzoic, gallic, 4-ethoxyphenylphosphonic, 4-methylphenylphosphonic, 4-chlorophenylphosphonic, phenylphosphonic acid or a mixture thereof.

4. The bar composition according to claim 3 wherein the acid is benzoic, p-amino benzoic, salicylic, capryloyl salicylic, 3-hydroxybenzoic, 4-hydroxybenzoic acid or a mixture thereof.

5. The bar composition according to claim 4 wherein the acid is benzoic or salicylic acid or a mixture thereof and the anionic surfactant is at least 90% by weight isethionate.

6. The bar composition according to any one of the preceding claims, wherein the bar composition comprises from 0.00001 to 3.0% or 0.0% by weight zwitterionic surfactant wherein the zwitterionic surfactant is a betaine or sultaine or a mixture thereof.

7. The bar composition according to claim 6, wherein betaine and/or sultaine are present and the bar composition comprises from 0.001 to 3.5% or 0.0% by weight taurate.

8. The bar composition according to claim any one of the preceding claims, wherein the bar composition comprises less than 9% by weight soap or 0.0% by weight soap and is substantially free of ethanol, a paraben, formaldehyde donor, silicone, sulfate, glycinate and/or halogenated antimicrobial active, substantially free meaning no more than 1.5% by weight of the total weight of the bar composition.

9. The bar composition according to any one of the preceding claims, wherein the bar composition is substantially free of silicone, sulfate, C₁₆₋₁₈ methyl ester sulphonate, glycinate, carboxylate, glutamates and/or alpha olefin sulfonate and has a zein value ≤10, substantially free meaning no more than 1.5% by weight of the total weight of the bar composition.

10. The bar composition according to any one of the preceding claims, further comprising 12-hydroxystearic acid, cetylpyridinium chloride, cetyltrimethylammonium chloride, cetyltrimethylammonium bromide, benzalkonium chloride, benzethonium chloride, cetrimide, certrimonium chloride and/or bromide, chloroxylenol, antimicrobial lipids, antimicrobial peptides, triclosan, thymol, terpineol or a mixture thereof.

11. The bar composition according to any one of the preceding claims, wherein the 10% aqueous slurry has a pH from 3.9 to 4.8.

12. A method for reducing both Gram negative and positive antimicrobial presence on a surface in need of antimicrobial reduction comprising the steps of:
a) contacting the surface with a bar slurry comprising:
i. from 60 to 99% by weight water at a temperature of from 32 to 46°C;
ii. from 1 to 40% by weight water soluble and insoluble components from the bar composition of any one of claims 1 to 11; and
b) rinsing the slurry from the surface
wherein soluble and insoluble components make up the total component weight in the slurry that comprises from 20 to 40% by weight soluble components in step a), and further wherein the bar composition has an antimicrobial activity in a 10% slurry that provides a Log₁₀ Reduction against *E. coli* ATCC 10536 and *S. aureus* ATCC 6538 of at least 2.75 after 30 seconds of contact, the bar slurry having a pH from 2.9 to 5.2.

13. The method according to claim 12, wherein the surface is hair, skin or an inanimate object.

## Patentansprüche

1. Stückförmige Zusammensetzung, umfassend:
a) 20 bis 60 Gew.-% anionisches Tensid;
b) 0,2 bis 8 Gew.-% mindestens einer Säure mit einem pKa-Wert von 1 bis 8 und einem log P-Wert von 0 bis 4,95;
c) strukturgebend für die Zusammensetzung Fettsäure, Polyalkylenoxid oder eine Mischung davon,
wobei die stückförmige Zusammensetzung in einer 10%-igen wässrigen Aufschlämmung einen pH-Wert von 2,9 bis 5,2 aufweist, mit der Maßgabe, dass, wenn die Aufschlämmung einen pH-Wert über 4,45 aufweist und die stückförmige Zusammensetzung 0,5 Gew.-% oder weniger mindestens einer Säure mit einem pKa-Wert von 1 bis 8 und einem log P-Wert von 0 bis 4,95 aufweist, die stückförmige Zusammensetzung weniger als 3 Gew.-% an insgesamt von Betain, Sultain abgeleitetem Tensid und Mischungen davon aufweist, und wobei die stückförmige Zusammensetzung ferner im Wesentlichen sulfatfrei ist, wobei im Wesentlichen frei nicht mehr als 1,5 Gew.-% des Gesamtgewichts der stückförmigen Zusammensetzung bedeutet,
wobei das anionische Tensid eine Mischung aus Taurat und Isethionat in einem Gewichtsverhältnis von 99:1 bis 1:99 umfasst und die Oberfläche Haut ist.

2. Stückförmige Zusammensetzung nach Anspruch 1, wobei die Säure einen pKa-Wert von 1,5 bis 7 oder von 2,5 bis 4,8 oder von 2,75 bis 3,25 und einen log P-Wert von 0,65 bis 4 oder von 1 bis 3,3 oder von 1,2 bis 3 aufweist.

3. Stückförmige Zusammensetzung nach irgendeinem der vorangehenden Ansprüche, wobei die Säure Propionsäure, n-Buttersäure, Isobuttersäure, Pentansäure, Hexansäure, Heptansäure, Octansäure, Nonansäure, Decansäure, Pimelinsäure, 3-Butensäure, 2-Butinsäure, Crotonsäure, Isocrotonsäure, Sorbinsäure, Angelinsäure, Tiglinsäure, Tetrolsäure, Cumarinsäure, Benzoesäure, p-Aminobenzoesäure, Salicylsäure, Capryloylsalicylsäure, 3-Hydroxybenzoesäure, 4-Hydroxbenzoesäure, Gallussäure, 4-Ethoxyphenylphosphonsäure, 4-Methylphenylphosphonsäure, 4-Chlorphenylphosphonsäure, Phenylphosphonsäure oder eine Mischung davon ist.

4. Stückförmige Zusammensetzung nach Anspruch 3, wobei die Säure Benzoesäure, p-Aminobenzoesäure, Salicylsäure, Capryloylsalicylsäure, 3-Hydroxybenzoesäure, 4-Hydroxybenzoesäure oder eine Mischung davon ist.

5. Stückförmige Zusammensetzung nach Anspruch 4, wobei die Säure Benzoesäure oder Salicylsäure oder eine Mischung davon ist und das anionische Tensid zu mindestens 90 Gew.-% Isethionat ist.

6. Stückförmige Zusammensetzung nach irgendeinem der vorhergehenden Ansprüche, wobei die stückförmige Zusammensetzung 0,00001 bis 3,0 Gew.-% oder 0,0 Gew.-% zwitterionisches Tensid umfasst, wobei das zwitterionische Tensid ein Betain oder Sultain oder eine Mischung davon ist.

7. Stückförmige Zusammensetzung nach Anspruch 6, wobei Betain und/oder Sultain vorhanden sind und die stückförmige Zusammensetzung 0,001 bis 3,5 Gew.-% oder 0,0 Gew.-% Taurat umfasst.

8. Stückförmige Zusammensetzung nach irgendeinem der vorhergehenden Ansprüche, wobei die stückförmige Zusammensetzung weniger als 9 Gew.-% Seife oder 0,0 Gew.-% Seife umfasst und im Wesentlichen frei von Ethanol, Paraben, Formaldehydspender, Silikon, Sulfat, Glycinat und/oder halogeniertem antimikrobiellem Wirkstoff ist, wobei im Wesentlichen frei nicht mehr als 1,5 Gew.-% des Gesamtgewichts der stückförmigen Zusammensetzung bedeutet.

9. Stückförmige Zusammensetzung nach irgendeinem der vorhergehenden Ansprüche, wobei die stückförmige Zusammensetzung im Wesentlichen frei von Silikon, Sulfat, C₁₆₋₁₈-Methylestersulfonat, Glycinat, Carboxylat, Glutamaten und/oder Alpha-Olefinsulfonat ist und einen Zein-Wert von ≤10 aufweist, wobei im Wesentlichen frei nicht mehr als 1,5 Gew.-% des Gesamtgewichts der stückförmigen Zusammensetzung bedeutet.

10. Stückförmige Zusammensetzung nach irgendeinem der vorhergehenden Ansprüche, ferner umfassend 12-Hydroxystearinsäure, Cetylpyridiniumchlorid, Cetyltrimethylammoniumchlorid, Cetyltrimethylammoniumbromid, Benzalkonoiumchlorid, Benzethoniumchlorid, Cetrimid, Cetrimoniumchlorid und/oder -bromid, Chloroxylenol, antimikrobielle Lipide, antimikrobielle Peptide, Triclosan, Thymol, Terpineol oder eine Mischung davon.

11. Stückförmige Zusammensetzung nach irgendeinem der vorhergehenden Ansprüche, wobei die 10%-ige wässrige Aufschlämmung einen pH-Wert von 3,9 bis 4,8 aufweist.

12. Verfahren zur Reduzierung sowohl gramnegativer als auch grampositiver antimikrobieller Präsenz auf einer Oberfläche, bei der eine antimikrobielle Reduzierung erforderlich ist, umfassend die folgenden Schritte:
a) Inkontaktbringen der Oberfläche mit einer Aufschlämmung einer stückförmigen Zusammensetzung, die umfasst:
i. 60 bis 99 Gew.-% Wasser bei einer Temperatur von 32 bis 46°C;
ii. 1 bis 40 Gew.-% wasserlösliche und wasserunlösliche Bestandteile aus der stückförmigen Zusammensetzung nach irgendeinem der Ansprüche 1 bis 11; und
b) Abspülen der Aufschlämmung von der Oberfläche,
wobei lösliche und unlösliche Bestandteile das Gesamtgewicht der Bestandteile in der Aufschlämmung ausmachen, die in Schritt a) 20 bis 40 Gew.-% lösliche Bestandteile umfasst, und wobei ferner die stückförmige Zusammensetzung in einer 10-prozentigen Aufschlämmung eine antimikrobielle Wirkung aufweist, die nach 30 Sekunden Kontakt eine Log₁₀-Reduktion gegen E. coli ATCC 10536 und S. aureus ATCC 6538 von mindestens 2,75 bewirkt, wobei die Aufschlämmung der stückförmigen Zusammensetzung einen pH-Wert von 2,9 bis 5,2 aufweist.

13. Verfahren nach Anspruch 12, wobei die Oberfläche Haar, Haut oder ein unbelebter Gegenstand ist.

## Revendications

1. Composition de pain comprenant :
a) de 20 à 60 % en poids de tensioactif anionique ;
b) de 0,2 à 8 % en poids d'au moins un acide ayant un pKa de 1 à 8 et un log P de 0 à 4,95 ;
c) un acide gras structurant pour pain, du poly(oxyde d'alkylène) ou un mélange de ceux-ci,
dans laquelle la composition de pain est une bouillie aqueuse à 10 % et a un pH de 2,9 à 5,2, sous réserve que, lorsque la bouillie a un pH supérieur à 4,45 et la composition de pain a 0,5 % en poids ou moins d'au moins un acide ayant un pKa de 1 à 8 et un log P de 0 à 4,95, la composition de pain ait moins de 3 % en poids au total de bétaïne, tensioactif dérivé de sultaïne et leurs mélanges, et en outre dans laquelle la composition de pain est pratiquement sans sulfates, "pratiquement sans" signifiant au plus 1,5 % en poids par rapport au poids total de la composition de pain,
dans laquelle le tensioactif anionique comprend un mélange de taurate et d'iséthionate en un rapport en poids de 99/1 à 1/99, et la surface est la peau.

2. Composition de pain selon la revendication 1, dans laquelle l'acide a un pKa de 1,5 à 7 ou de 2,5 à 4,8 ou de 2,75 à 3,25 et un log P de 0,65 à 4 ou de 1 à 3,3 ou de 1,2 à 3.

3. Composition de pain selon l'une quelconque des revendications précédentes, dans laquelle l'acide est l'acide propionique, n-butyrique, isobutyrique, pentanoïque, hexanoïque, heptanoïque, octanoïque, nonanoïque, décanoïque, pimélique, but-3-énoïque, but-2-ynoïque, crotonique, isocrotonique, sorbique, angélique, tiglique, tétrolique, coumarique, benzoïque, p-aminobenzoïque, salicylique, capryloylsalicylique, 3-hydroxybenzoïque, 4-hydroxybenzoïque, gallique, 4-éthoxyphénylphosphonique, 4-méthylphénylphosphonique, 4-chlorophénylphosphonique, phénylphosphonique ou un mélange de tels acides.

4. Composition de pain selon la revendication 3, dans laquelle l'acide est l'acide benzoïque, p-aminobenzoïque, salicylique, capryloylsalicylique, 3-hydroxybenzoïque, 4-hydroxybenzoïque ou un mélange de tels acides.

5. Composition de pain selon la revendication 4, dans laquelle l'acide est l'acide benzoïque ou salicylique ou un mélange de tels acides et le tensioactif anionique est, à au moins 90 % en poids, un iséthionate.

6. Composition de pain selon l'une quelconque des revendications précédentes, dans laquelle la composition de pain comprend de 0,00001 à 3,0 % ou 0,0 % en poids de tensioactif zwittérionique, dans laquelle le tensioactif zwittérionique est une bétaïne ou sultaïne ou un mélange de celles-ci.

7. Composition de pain selon la revendication 6, dans laquelle les bétaïne et/ou sultaïne sont présentes et la composition de pain comprend de 0,001 à 3,5 % ou 0,0 % en poids de taurate.

8. Composition de pain selon l'une quelconque des revendications précédentes, dans laquelle la composition de pain comprend moins de 9 % en poids de savon ou 0,0 % en poids de savon et est pratiquement sans éthanol, paraben, donneur de formaldéhyde, silicone, sulfate, glycinate et/ou agent actif antimicrobien halogéné, "pratiquement sans" signifiant au plus 1,5 % en poids par rapport au poids total de la composition de pain.

9. Composition de pain selon l'une quelconque des revendications précédentes, dans laquelle la composition de pain est pratiquement sans silicone, sulfate, sulfonate d'ester méthylique en C₁₆ à C₁₈, glycinate, carboxylate, glutamates et/ou alpha-oléfinesulfonate et a une valeur de zéine ≤ 10, "pratiquement sans" signifiant au plus 1,5 % en poids par rapport au poids total de la composition de pain.

10. Composition de pain selon l'une quelconque des revendications précédentes, comprenant en outre de l'acide 12-hydroxystéarique, du chlorure de cétylpyridinium, du chlorure de cétyltriméthylammonium, du bromure de cétyltriméthylammonium, du chlorure de benzalkonium, du chlorure de benzéthonium, du cétrimide, du chlorure et/ou bromure de cétrimonium, du chloroxylénol, des lipides antimicrobiens, des peptides antimicrobiens, du triclosan, du thymol, du terpinéol ou un mélange de ceux-ci.

11. Composition de pain selon l'une quelconque des revendications précédentes, dans laquelle la bouillie aqueuse à 10 % a un pH de 3,9 à 4,8.

12. Procédé pour réduire la présence antimicrobienne Gram tant négative que positive sur une surface nécessitant une réduction antimicrobienne, comprenant les étapes de :
a) mise en contact de la surface avec une bouillie de pain comprenant :
i. de 60 à 99 % en poids d'eau à une température de 32 à 46 °C ;
ii. de 1 à 40 % en poids de composants solubles et insolubles dans l'eau provenant de la composition de pain de l'une quelconque des revendications 1 à 11 ; et
b) rinçage de la bouillie sur la surface
dans lequel les composants solubles et insolubles constituent le poids total des composants dans la bouillie qui comprend de 20 à 40 % en poids de composants solubles dans l'étape a), et en outre dans lequel la composition de pain a une activité antimicrobienne dans une bouillie à 10 % qui assure une réduction Log₁₀ contre E. coli ATCC 10536 et S. aureus ATCC 6538 d'au moins 2,75 après 30 secondes de contact, la bouillie de pain ayant un pH de 2,9 à 5,2.

13. Procédé selon la revendication 12, dans lequel la surface est une chevelure, de la peau ou un objet inanimé.
